# EUROPEAN PATENT SPECIFICATION

(11) **EP 3 580 356 B1**
(45) Date of publication and mention of the grant of the patent: **31.03.2021**
(21) Application number: 18706005.8
(22) Date of filing: 09.02.2018
(51) Int. Cl.: C12Q 1/6804, C12N 15/10

(54) **METHOD FOR DETERMINING LEVELS OF INTERACTIONS BETWEEN BIOMOLECULES**
VERFAHREN ZUR BESTIMMUNG VON INTERAKTIONEN ZWISCHEN BIOMOLEKÜLEN
PROCÉDÉ DE DÉTERMINATION DE NIVEAUX D'INTERACTIONS ENTRE DES BIOMOLÉCULES

(30) Priority: 09.02.2017 SE 1750122
(43) Date of publication of application: 18.12.2019
(73) Proprietor: Atlas Antibodies AB, 168 69 Bromma (SE)
(72) Inventor: SÖDERBERG, Ola, 74891 Österbybruk (SE)
(74) Representative: Kransell & Wennborg KB
(86) International application number: PCT/SE2018/050121
(87) International publication number: WO 2018/147794

(56) References cited:
- WO-A1-2015/118029
- WO-A2-2006/137932
- K.-J. LEUCHOWIUS ET AL: "Parallel Visualization of Multiple Protein Complexes in Individual Cells in Tumor Tissue", MOLECULAR & CELLULAR PROTEOMICS, vol. 12, no. 6, 22 February 2013 (2013-02-22), pages 1563-1571, XP055289149, US ISSN: 1535-9476, DOI: 10.1074/mcp.O112.023374 -& Leuchowius ET AL: "Parallel Visualization of Multiple Protein Complexes in Individual Cells in Tumor Tissue: Supplemental Fig 15", , 22 February 2013 (2013-02-22), XP055463786, Retrieved from the Internet: URL:http://www.mcponline.org/content/suppl /2013/02/22/O112.023374.DC1/Suppl_fig_15.p df [retrieved on 2018-03-29]

### SEQUENCE LISTING

<110> Söderberg, Ola
<120> Method for analysis of protein-protein interactions
<130> P41604751PCT00
<160> 30
<170> PatentIn version 3.5
<210> 1
   <211> 92
   <212> DNA
   <213> synthetic
<400> 1
   cgaggtgctt ttagcacctc gaagtaaagc ccgtcccagt gaatgcgagt ccgtctgata 60
   acctagataa acgtcacact tttcgtgtga cg 92
<210> 2
   <211> 94
   <212> DNA
   <213> synthetic
<400> 2
   tttatctata tccctacttc acctgccucg tctattccac ctcaaaaagt gtccactcct 60
   accuctgccc actacctacc tcaaaccttt actt 94
<210> 3
   <211> 25
   <212> DNA
   <213> synthetic
<400> 3
   tctgcagtta tacgtccaat cataa 25
<210> 4
   <211> 25
   <212> DNA
   <213> synthetic
<400> 4
   tcgtacgtag atcctgccat ttcta 25
<210> 5
   <211> 61
   <212> DNA
   <213> synthetic
<220>
   <221> misc_feature
   <223> 5'-end substituted with aldehyde
<400> 5
   aaaaaggtag gtagtgggca gttatgattg gacgtataac tgcagaggta ggagtggaca 60
   c 61
<210> 6
   <211> 60
   <212> DNA
   <213> synthetic
<220>
   <221> misc_feature
   <223> 5'-end substituted with aldehyde
<400> 6
   aaaaagaggt ggaatagacg tagaaatggc aggatctacg tacgaggcag gtgaagtagg 60
<210> 7
   <211> 29
   <212> DNA
   <213> synthetic
<400> 7
   taggtagtgg gcagaggtag gagtggaca 29
<210> 8
   <211> 29
   <212> DNA
   <213> synthetic
<400> 8
   aggtggaata gacgaggcag gtgaagtag 29
<210> 9
   <211> 93
   <212> DNA
   <213> synthetic
<400> 9
   tttatctata tccctactta cgtctctcgt ctattccacc tcaaaaagtg tccactcgtc 60
   tcactgccca ctacctacct caaaccttta ctt 93
<210> 10
   <211> 26
   <212> DNA
   <213> synthetic
<400> 10
   ctgcgcagtt atacgtccaa tcataa 26
<210> 11
   <211> 24
   <212> DNA
   <213> synthetic
<400> 11
   cgtacgtaga tcctgccatt tcta 24
<210> 12
   <211> 58
   <212> DNA
   <213> synthetic
<220>
   <221> misc_feature
   <223> 5'-end substituted with aldehyde
<400> 12
   ggtaggtagt gggcagttat gattggacgt ataactgcgc agtgagacga gtggacac 58
<210> 13
   <211> 54
   <212> DNA
   <213> synthetic
<220>
   <221> misc_feature
   <223> 5'-end substituted with aldehyde
<400> 13
   gaggtggaat agacgtagaa atggcaggat ctacgtacga gagacgtaag tagg 54
<210> 14
   <211> 23
   <212> DNA
   <213> synthetic
<220>
   <221> misc_feature
   <223> 5'-end substituted with Cy3
<400> 14
   tctgcagtta tacgtccaat uuu 23
<210> 15
   <211> 23
   <212> DNA
   <213> synthetic
<220>
   <221> misc_feature
   <223> 5'-end substituted with FITC
<400> 15
   tcgtacgtag atcctgccat uuu 23
<210> 16
   <211> 112
   <212> DNA
   <213> synthetic
<400> 16
   tttatctata tccctacttc acctgcctcg tacgtagauc tattccacct ctctaaaaaa 60
   aatccactcc tacctctgta gttauaccta cctcgtgagg aaacctttac tt 112
<210> 17
   <211> 27
   <212> DNA
   <213> synthetic
<400> 17
   tcacgtccaa tcgataacta cagttat 27
<210> 18
   <211> 25
   <212> DNA
   <213> synthetic
<400> 18
   ttcctgccat tatctacgtg tagat 25
<210> 19
   <211> 66
   <212> DNA
   <213> synthetic
<220>
   <221> misc_feature
   <223> 5'-end substituted with aldehyde
<400> 19
   aacctcacga ggtaggtata actgtagtta tcgattggac gtgataacta cagaggtagg 60
   agtgga 66
<210> 20
   <211> 65
   <212> DNA
   <213> synthetic
<220>
   <221> misc_feature
   <223> 5'-end substituted with aldehyde
<400> 20
   aatagagagg tggaatagat ctacacgtag ataatggcag gaatctacgt acgaggcagg 60
   tgaag 65
<210> 21
   <211> 109
   <212> DNA
   <213> synthetic
<400> 21
   tttatctata tccctacttc acctgcctcg tacgtagatc attgcacctc tctaaaaaat 60
   ccactcctac ctctgtagtt atcattgcct cgtgaggaaa cctttactt 109
<210> 22
   <211> 26
   <212> DNA
   <213> synthetic
<400> 22
   cacgtccaat cgataactac agttat 26
<210> 23
   <211> 24
   <212> DNA
   <213> synthetic
<400> 23
   tcctgccatt atctacgtgt agat 24
<210> 24
   <211> 64
   <212> DNA
   <213> synthetic
<220>
   <221> misc_feature
   <223> 5'-end substituted with aldehyde
<400> 24
   cctcacgagg caatgataac tgtagttatc gattggacgt gataactaca gaggtaggag 60
   tgga 64
<210> 25
   <211> 62
   <212> DNA
   <213> synthetic
<220>
   <221> misc_feature
   <223> 5'-end substituted with aldehyde
<400> 25
   tagagaggtg caatgatcta cacgtagata atggcaggaa tctacgtacg aggcaggtga 60
   ag 62
<210> 26
   <211> 22
   <212> DNA
   <213> synthetic
<220>
   <221> misc_feature
   <223> 5'-end substituted with Cy3
<400> 26
   tccaatcgat aactacagtt at 22
<210> 27
   <211> 21
   <212> DNA
   <213> synthetic
<220>
   <221> misc_feature
   <223> 5'-end substituted with FITC
<400> 27
   tgccattatc tacgtgtaga t 21
<210> 28
   <211> 96
   <212> DNA
   <213> synthetic
<220>
   <221> misc_feature
   <223> 5'-end substituted with aldehyde; fluorophore alexa 555 at position T19; quencher BHQ3 at position A43
<400> 28
   aaaaaaaaaa ggtcctgatc cactcctact tccactccca tcaggtgaag tgaagtaggt 60
   aagtgatggg agtggaagta ggagtggatc aggacc 96
<210> 29
   <211> 72
   <212> DNA
   <213> synthetic
<220>
   <221> misc_feature
   <223> Fluorophore Alexa 488 at position T1; fluorophore alexa 647 at position T29; Quencher BHQ1 at postion T55; 3'-end substituted with aldehyde
<400> 29
   tccacttcta tttccactcc catcatcggt gatgggagtg gaagtaggag tggatcagga 60
   ccaaaaaaaa aa 72
<210> 30
   <211> 39
   <212> DNA
   <213> synthetic
<400> 30
   cctacttcca ctcccatcac ttacctactt cacttcacc 39

## Description

### Technical field

The present invention relates to a method for determining levels of interactions between biomolecules, such as proteins, in a sample. Also, the invention refers to kits for use in the method of the invention.

### Technical background

Methods to determine levels of protein-protein interactions are essential in analysis of cellular signaling activity. Over the years a multitude of methods have been developed to facilitate this. Several such methods are based on genetic constructs, where candidate proteins are fused with reporter molecules that upon an interaction will reconstitute a functional reporter, i.e. protein fragment complementation assays. Examples of such methods are Yeast-two-hybrid (Y2H) (Fields et al., 1989, Nature 340(6230): 245-246), mammalian-membrane two-hybrid (MaMTH) (Petschnigg et al. 2014, Nat Methods 11(5): 585-592) and bimolecular fluorescence complementation (BiFC) (Hu et al. 2002, Mol Cell 9(4): 789-798). Another common approach is to use Förster resonance energy transfer (FRET) to determine binding of fluorescent fusion proteins, with a concomitant change in emission. To determine interactions between native proteins there are several methods based on antibodies to target the proteins, where the antibodies are conjugated with functional groups to confer isolation or detection of protein complexes, such as co-immunoprecipitation antibody-based FRET or Proximity Ligation Assay (PLA).

Proximity Ligation Assay (PLA) (e.g. WO2009/021031) is based upon antibodies conjugated with oligonucleotides, called proximity probes, that template the ligation of two subsequently added circularization oligonucleotides into a circular molecule. Only if a pair of proximity probes binds adjacent epitopes the creation of a circular reporter molecule will be allowed. The oligonucleotide on one of the proximity probes will then prime a rolling circle amplification (RCA). Single proximity probes will template the ligation of a linear molecule, which cannot be amplified by RCA. Hence only proximity events, such as protein-protein interactions will be detected.

In WO2015/118029, a proximity assay with detection based on hybridisation chain reaction is disclosed.

Leuchowius et al ("Parallel visualization of multiple protein complexes in individual cells in tumor tissue", Molecular & Cellular Proteomics, 2013, June; 12(6): 1563-1571) discloses a multiplex proximity ligation assay for simultaneous visualization of multiple protein complexes in situ.

WO2006/137932 relates to novel binding pair compositions of defined and limited stability comprising nucleic acid detection markers for homogenous and sensitive detection of analytes.

### Summary of invention

Although PLA and other known proximity assays are sensitive and selective methods for detecting protein-protein interactions, it is not possible to determine how large proportion of a pool of a protein is actually involved in an interaction.

The two method aspects described herein provide information on both interacting and free proteins. One of the designs provide signal amplification to detect single molecules, using DNA polymerase to amplify a DNA oligonucleotide that has received information on if proteins interact or not, while the other only are based on DNA hybridization to position fluorophores and quenchers so that they report in different colors if the proteins are free or interacting with eachother.

Thus, generally, the invention relates to a method for determining levels of interactions between biomolecules, such as proteins, in a sample, comprising providing a first and a second information carrying (IC) oligonucleotide, wherein the first and second IC oligonucleotide are attached, covalently or non-covalently, to a first and a second affinity reagent, such as antibodies, that have the capacity to bind to a first and a second biomolecule, wherein the first and second IC oligonucleotide each comprises at least one single-stranded stretch that is complementary to a part of another oligonucleotide, thereby, upon hybridisation of the at least one single-stranded stretch in at least one of the first and second IC oligonucleotides to its complementary part of another oligonucleotide, enabling measurement of the relative proportion of interacting and non-interacting first and second biomolecules in the sample at a single cell or single molecular level.

By "covalent or non-covalent attachment" is in this context meant that the affinity reagent(s), such as antibodies, is attached to the IC oligonucleotide by means of any kind of chemical binding, as long as the binding is strong enough to allow the interaction between the biomolecules to be analyzed.

By an "affinity reagent" is typically meant an antibody or a reagent comprising an antibody, even though other types of molecules can be used as long as the "affinity reagent" has the capacity to bind to the biomolecule to be analyzed and thereby fulfil the purpose of the invention. "Other types of molecules" could e.g. be any type of biomolecule, such as a nucleic acid molecule, a polypeptide or any other kind, having the capacity to bind to the biomolecule to be analyzed.

By an "IC probe" or "probe" is meant an IC oligonucleotide bound to an affinity reagent.

By "measurement of the relative proportion" interacting and non-interacting biomolecules is meant that the relative amounts of biomolecules that bind to each other and do not bind to each other can be measured, which is an important purpose of the invention.

By "single cell or single molecular level" is meant that the measurement of interaction and non-interaction between biomolecules can be performed for single cells or single molecules, i.e. improved read-out properties compared to prior art methods.

"The single-stranded stretch" of the first and second IC oligonucleotides enables interaction (a) between the first and/or second IC oligonucleotide and an information receiving (IR) oligonucleotide, (b) between the first and/or second IC oligonucleotide and an activating oligonucleotide, or (c) directly between the first and second IC oligonucleotide. The single-stranded stretch must have a length that is sufficient to allow hybridisation of the single-stranded stretch to a complementary part of another oligonucleotide.

By "activating oligonucleotide" is meant an oligonucleotide that upon binding to an IC oligonucleotide can cause restructuring of the intraolecular hybridization in the IC oligonucleotide, and thereby revealing a stretch of oligonucleotides complementary to another IC oligonucleotide, enabling hybridization between two different IC oligonucleotides that will result in repositioning of fluorophores and quenchers.

To be able to detect both interacting proteins and the pool of non-interacting proteins the inventors of the present invention developed a first aspect of the method, that can visualize interactions between protein A and protein B at the same time as it reports amounts of non-interacting protein A or non-interacting protein B at a single cell or single molecule level. According to this aspect of the method of the invention, oligonucleotides carrying the information on the identity of the proteins or biomolecules to be analyzed (information carrier (IC)) are provided. To these a preformed single-stranded DNA molecule (information receiver (IR)), that may be circular, is hybridized and cut open at areas where they are double stranded, i.e. where the IR molecule is hybridized to the IC molecule. Upon cleavage a short oligonucleotide tag, complementary to the IC molecule, will be incorporated into the IR molecule. The now re-created IR molecule can be amplified by RCA (if circular) or PCR (if linear) and the identity of the incorporated tags will be visualized by e.g. hybridization of fluorophore-labeled detection oligonucleotides, wherein the incorporation of one or two tags will provide information about the interaction between the proteins or biomolecules to be analyzed, as well as the relative amounts of free and interacting biomolecules or proteins. This method for molecular Boolean (MolBoolean) analysis provides a unique tool for determining the relation of free and interacting proteins, which is a requirement for mathematical modeling of signaling pathway activity.

Thus, in a first aspect the method of the invention comprises the steps of:
a. providing the single-stranded information receiving (IR) DNA molecule, wherein the IR DNA molecule is circular or linear, carrying at least a first and a second cleavage motif, wherein the cleavage motifs are chosen so that the cleavage motif sites must become double-stranded in order to allow cleavage;
b. providing the first information carrying (IC) DNA molecule, comprising a single-stranded stretch that is complementary to the part of the IR DNA molecule carrying the first cleavage motif, wherein the occurrence of the first IC DNA molecule reflects the amount of a first biomolecule in the sample;
c. providing the second information carrying (IC) DNA molecule, comprising a single-stranded stretch that is complementary to the part of the IR DNA molecule carrying the second cleavage motif, wherein the occurrence of the second IC DNA molecule reflects the amount of a second biomolecule in the sample;
d. mixing the DNA molecules of step a-c under conditions that allow binding of complementary single-stranded stretches;
e. adding digestion enzyme(s) to create nick(s) at the cleavage motif site(s) that has/have become double-stranded, thereby forming
   i. a first reporter tag binding site that will allow binding of a first reporter tag DNA molecule or sequence, comprising a stretch that is complementary to a part of the first IC DNA molecule, and/or
   ii. a second reporter tag binding site that will allow binding of a second reporter tag DNA molecule or sequence comprising a stretch that that is complementary to a part of the second IC DNA molecule;
f. incorporating reporter tag DNA sequences by any one of the following alternatives:
   i. adding the first reporter tag DNA molecule and the second reporter tag DNA molecule, whereby the first reporter tag DNA molecule is incorporated in the IR DNA molecule at the first reporter tag binding site if a nick has been created at the first cleavage motif site, and/or the second reporter tag DNA molecule is incorporated in the IR DNA molecule at the second reporter tag binding site if a nick has been created at the second cleavage motif site; or
   ii. using a DNA polymerase and nucleotides to incorporate the first reporter tag DNA sequence in the IR DNA molecule by filling the gap complementary to the first reporter tag binding site on the first IC oligonucleotide if a nick has been created at the first cleavage motif site, and/or to incorporate the second reporter tag DNA sequence in the IR DNA molecule by filling the gap complementary to the second reporter tag binding site on the second IC oligonucleotide if a nick has been created at the second cleavage motif site; and
      adding a ligation enzyme ligating the IR DNA molecule, thereby providing a recreated IR DNA molecule;
g. optionally amplifying the recreated IR DNA molecule of step f;
h. monitoring the incorporation of the first and/or second reporter tag(s) in the recreated IR DNA molecule, as a measurement of the occurrence of and/or interaction between the first and/or the second biomolecule(s) in the sample.

The IR DNA molecule can be linear or circular. It is important that the IR DNA molecule has the ability to be cut when it binds to an IC DNA molecule and it needs to be partially complementary to the IC DNA molecule.

The IC DNA molecules must be partially complementary to the IR DNA molecule and includes additional DNA bases that will template the insertion of the complement sequence into the cleaved IR DNA molecule.

By "reflecting the amount" of a biomolecule is meant that the relative amount or occurrence of the IC DNA molecule, and its subsequent hybridisation/binding to the IR DNA molecule, is a measurement of the relative occurrence or amount of free and interacting biomolecule in the sample. Hence, the relative amounts of the first and the second IC DNA molecules are a relative measurement of the amount or occurrence of free and interacting first and second biomolecules, respectively, in the sample.

By "conditions allowing binding of complementary single-stranded stretches" are meant such conditions that typically are referred to as allowing stringent hybridisation. A skilled person in the art would know and/or would easily find out suitable conditions for the actual hybridisation reactions.

By a "cleavage motif" is, in the context of the present invention, meant a motif or short sequence of the DNA molecule that a restriction enzyme or the like can recognise. The restriction enzyme or the like recognising a cleavage motif can then bind to the site of the cleavage motif (i.e. the "cleavage motif site") and, under certain conditions, cleave, or "create a nick" to, one strand of the double-stranded DNA molecule, so that a binding site for a short reporter tag DNA molecule is created, i.e. a "reporter tag binding site".

Many possible cleavage motifs can be used, as long as they allow cleaving only when the motif has become double-stranded. Preferred cleavage motifs are chosen from uracils or restriction sites.

Further, the digestion enzyme(s) used to create nicks at the cleavage motif sites are preferably chosen from
i. nicking endonuclease , e.g. Nb.Bsr.DI or Nt.BsmAI nicking a specific strand in double stranded restriction site, or
ii. a combination of uracil-DNA glycolsylase (UDG), removing a uracil base at a double stranded site, and EndolV, removing the apyrimidinic site.

Other enzyme or enzyme combinations are also fully possible e.g. restriction enzymes, enzymes used in DNA repair such as MutY, or engineered restriction enzymes such as Transcription activator-like effector nucleases (Talen) as along as the chosen enzyme or enzyme combination allows cleaving one DNA strand at a double stranded site.

In one embodiment, the first IC DNA molecule is conjugated to a first antibody molecule being equal to or targeting the first biomolecule, and the second IC DNA molecule is conjugated to the second antibody molecule being equal to or targeting the second biomolecule.

The reporter tag DNA molecule is a short DNA molecule that is complementary to part of the IC DNA molecule.

The reporter tag DNA sequence is created by using DNA polymerase to add nucleotides, as an alternative to provide a reporter tag DNA molecule (see step f(i) and f (ii), respectively.

Thus, an alternative approach to transfer the sequence information to the IR molecule is to use DNA polymerases to add the nucleotides, templated by the nucleotides of the IC, to seal the gap formed by nicking the IR-IC hybrid.

The ligation enzyme(s) can e.g. be chosen from DNA ligase, or any other alternative.

The amplification step of the method can e.g. be performed by RCA (rolling circle amplification) for circular IR DNA molecules or PCR (polymerase chain reaction) for linear IR DNA molecules, or any other commonly used method. The skilled person in the art would easily know suitable amplification methods.

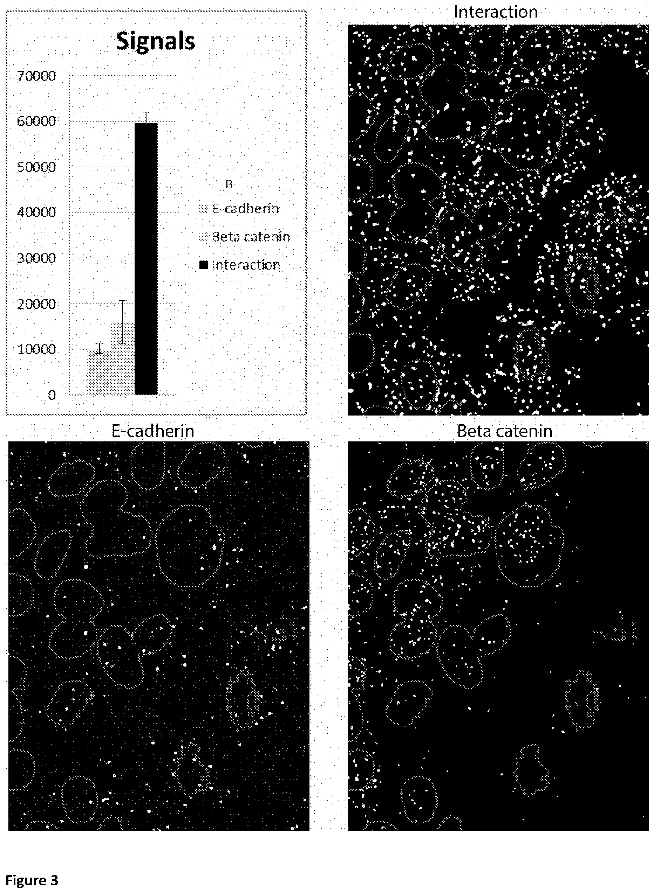

The monitoring step of the method of the invention can be performed by
a. providing a first labeled detection oligonucleotide that is complementary to at least a part of the first reporter tag DNA molecule that has been incorporated into the IR, and a second labeled detection oligonucleotide that is complementary to at least a part of the second reporter tag DNA molecule that has been incorporated into the IR, and
b. hybridizing the first and second labeled detection oligonucleotides to the recreated IR DNA molecule, which optionally is amplified,
wherein the labels of the first and second detection oligonucleotides are chosen from fluorophores having different read-out wavelengths, fluorophores in combination with quenchers **(****Figure 3****),** enzymes (e.g. horseradish peroxidase and alkaline phosphatase that can convert a substrate to a coloured precipitate), and molecules with different masses (the mass-tags can be recorded by a mass spectrometer). The labels can of course be chosen differently as long as they contain a specific property that can be measured and recorded.

In one embodiment a linear recreated IR DNA molecule is amplified and thereafter separated by a separation method, such as electrophoresis, especially gel electrophoresis, or chromatography, wherein separation products having different sizes indicate incorporation of different reporter tags.

In another embodiment, the identities of different reporter tags are monitored by sequencing.

In yet another embodiment, the recreated IR DNA molecule is monitored in the sample where they have been formed, such as by microscopy, or wherein the recreated IR DNA molecules are collected from the sample where they have been formed followed by sorting and analysis of single molecules, such as by microscopy.

In a further embodiment oligonucleotides are conjugated to antibodies, where the oligonucleotides carry the information on the identity of each antibody (information carrier (IC)). To these conjugates a preformed single-stranded DNA circle (information receiver (IR)) is hybridized and cut open at areas where they are double stranded, i.e. where the DNA circle is hybridized to an antibody-oligonucleotide conjugate. Upon cleavage, a short oligonucleotide tag, complementary to the antibody-oligonucleotide conjugate, will be incorporated into the circle which thereafter is ligated. The now re-created circle will be amplified by RCA and the identity of the incorporated tags will be visualized by hybridization of fluorophore-labeled detection oligonucleotides. If the RCA product is generated from a circle where only one tag is incorporated it will be fluorescent in only one wavelength, but if two tags are incorporated it will be labeled with two different fluorophores.

In yet another embodiment abasic sites are removed in order to improve the detection efficiency. This can be performed after the reporter tag molecules have been added and the first and/or second reporter tag have/has been incorporated into the IR, and the following steps are performed:
i. hybridizing a digestion template to the IR DNA molecule making the area around the remaining abasic site double stranded;
ii. digesting the double stranded area by a suitable restriction enzyme, such as EndolV;
iii. gap-filling the digested area with a suitable polymerase, such as T4 DNA polymerase, to add the missing base, such as thymidine; and ligating the IR DNA molecule with a ligase, such as T4 ligase, to provide a recreated IR DNA molecule.

Removing the abasic site(s) and ligating the circular molecule in this way, will improve the detection efficiency for IR DNA molecules with remaining abasic (apurinic or apyrimidinic) sites after reporter tag incorporation. By "improved detection efficiency" is meant that more amplification products will be generated for IR DNA molecules wherein abasic sites have been removed.

The "digestion template" is typically an oligonucleotide having a length that is sufficient to hybridise to the IR DNA molecule, also after removal of the abasic site. Typically, the length is about 20-30 nucleotides, but variations may occur as long as the digestion template hybridises to the IR DNA molecule under conditions allowing hybridization.

This embodiment is especially useful for situations where the motifs for cleavage in the IR DNA molecule are uracils, whereby a thymidine is added in the gap-fill process.

After performing these steps, a recreated IR DNA molecule is obtained, that optionally can be amplified, whereafter incorporation of the first and/or second reporter tag(s) is/are monitored.

In a second aspect, the invention relates to a method wherein:
a. the first and second IC oligonucleotides comprise hairpin structures in which fluorophores and quenchers are positioned, wherein the hairpin structures are designed so that only one fluorophore per oligonucleotide can emit light, and wherein each fluorophore has a unique signal;
b. the hairpins in the first and second IC oligonucleotides are disrupted or destabilized by either
   i. providing an activating oligonucleotide that is complementary to the first or the second IC oligonucleotide thereby binding to the first or second IC oligonucleotide, or
   ii. degrading one of the strands in the hairpins of one or both IC oligonucleotides, thereby liberating a single-stranded stretch of DNA in the first and/or second IC oligonucleotide,
   so that the first and second IC oligonucleotides can interact with each other causing a repositioning of fluorophores and quenchers; and
c. pairs of conjugates, comprising affinity reagents coupled to the first or second IC oligonucleotide, are used to interrogate proximity between two biomolecules to which the affinity reagents bind, wherein a first fluorophore signal pattern will be exhibited upon interaction between the first and second biomolecule, and a second fluorophore signal pattern will be exhibited upon lack of interaction between the first and second biomolecule, as a result of the oligonucleotides hybridizing to each other upon interaction between the first and second biomolecule causing restructuring of the positions of fluorophores and quenchers.

By "hairpin structure" is meant a section of the oligonucleotide where two stretches of the sequence are complementary to each other and thereby hybridize to each other leaving a stretch of unhybridized sequence inbetween, so that the molecule in this section forms a hairpin like structure.

By "the hairpins of the IC oligonucleotides" are "disrupted or destabilized" means that the section of the oligonucleotide forming a hairpin is disrupted so that the hairpin is dissolved and some other structure of the oligonucleotide is formed.

By "degrading one of the strands in the hairpins" means that the nucleotide sequence structure of at least part of the hairpin section is dissolved.

By "liberating" a single-stranded stretch of an IC oligonucleotide means that the liberated stretch no longer binds to a complementary part of e.g. a hairpin structure, and instead can, at least partly, hybridize to another oligonucleotide or section of the same oligonucleotide.

By "IC oligonucleotides interacting with each other" means that single-stranded stretches of some part of the oligonucleotides hybridizes to each other, causing "repositioning" and/or "restructuring of positions" of the fluorophores and quenchers within the IC oligonucleotides.

By "conjugates" are meant a combination of affinity reagent and IC oligonucleotide, wherein the affinity reagent is covalently or non-covalently bound to the IC oligonucleotide. By "pairs of conjugates" is accordingly meant two IC oligonucleotides, each bound to an affinity reagent, whereby the affinity reagents may interact with each other.

By "interrogate proximity" is meant that the proximity and/or interaction between the affinity reagents is monitored.

By "a fluorophore signal pattern" is meant that fluorophore signals (e.g. one or two fluorophore signals of different wavelengths) are readable, and that this signal pattern is unique for a certain interaction/structure between the IC oligonucleotides, whereas another interaction/structure between the IC oligonucleotides has another "fluorophore signal pattern".

This aspect is based on two, or more, oligonucleotides that are modified with fluorophores and quenchers, which are positioned so that only one fluorophore per oligonucleotide can emit light. These oligonucleotides can be activated, so upon proximal binding of a pair of antibodies, conjugated to such oligonucleotides, the oligonucleotides can hybridize to each other. Thereby repositioning the fluorophores and quenchers, so that the ones that previously could emit light now are quenched, and revealing a new fluorophore that can emit light (i.e. reporting on a protein-protein interaction).

Hereby, with the two presented aspects, a method is provided that solves the technical challenges of the prior art, and that offers improved read-out properties compared to prior art methods by providing information on both free and complex-bound proteins at a single cell level. Prior art methods used for detecting protein-protein interactions, such as PLA or Y2H gives information on levels of interactions but not on levels of non-interacting proteins. It has hence not been possible to determine if differences in levels of interactions recorded is due to different expression levels of the interacting proteins or if an interaction is regulated by e.g. post-translational modifications of the protein. By retrieving information on both expression levels of the proteins and the proportion that is participating in a protein complex, consisting of these proteins, it will be possible to visualize changes in dissociation constants - reflecting conformational changes of the proteins that are caused by e.g. post-translational modifications - in single cells. The method of the invention will facilitate the study and the understanding of cell signalling activities, and hence provides a novel research tool with broad clinical application.

The first and the second biomolecules of the method of the invention can e.g. be proteins or polypeptides, even though other biomolecules also can be monitored, such as DNA, RNA, carbohydrates, lipids, antibodies or any other type of biomolecule.

Typically, the sample is a biological sample, such as one or more cells, or a mixture of proteins. The method of the present invention may be used in many applications. In a preferred embodiment, the method is used for analysing protein-protein interactions e.g. in single cells, tissue sections, in body fluids or protein extracts.

In a third aspect, the present invention relates to a kit for use in the method of the first aspect of the invention, comprising
a. a single-stranded information receiving (IR) DNA molecule, carrying a first and a second cleavage motif;
b. a first and a second information carrying (IC) DNA molecule, reflecting the amounts of a first and a second biomolecule in a sample, wherein the first and second IC DNA molecules are conjugated to affinity reagents, or are provided with chemical moieties to be used for conjugation by a kit user;
c. optionally enzymes for creating nicks at the cleavage motif sites in the IR DNA molecule;
d. a first and a second reporter tag DNA molecule; and
e. optionally reagents for amplification of a recreated IR DNA molecule;
f. optionally a first and a second labelled detection oligonucleotide; and
g. optionally DNA molecule(s) and enzymes to remove abasic sites.

By "DNA molecule(s) and enzymes to remove abasic sites" are e.g. meant the digestion templates, as defiend above, as well as a suitable restriction enzyme, polymerase, and/or ligase, as discussed above in the embodiment relating to removal of abasic site(s).

By "a chemical moiety to be used for conjugation" is meant that the IC DNA molecules are prepared with a chemical part that in a later stage can be used for conjugation to an affinity reagent. Such "chemical moiety" could e.g. be chosen from biotin, streptavidin, avidin, NHS-ester, malemide, hydrazone, aldehyde, alkyne, azide, thiol, amine or any other suitable chemical moiety. The skilled person would be aware of other possible moieties.

In a fourth aspect, the invention relates to a kit for use in the method of the second aspect of the invention, comprising
a. a first and a second information carrying (IC) DNA molecule comprising hairpin structures in which fluorophores and quenchers are positioned, wherein the hairpin structures are designed so that only one fluorophore per oligonucleotide can emit light, and wherein each fluorophore has a unique signal, thereby having the ability to reflect the amounts of a first and a second biomolecule in a sample, wherein the first and second IC DNA molecules are conjugated to affinity reagents, such as antibodies, or are provided with chemical moieties to be conjugated by a kit user, and
b. an activating oligonucleotide that is complementary to the first or the second IC DNA molecule thereby having the ability to bind to the first or second IC DNA molecule.

### Brief description of the drawings

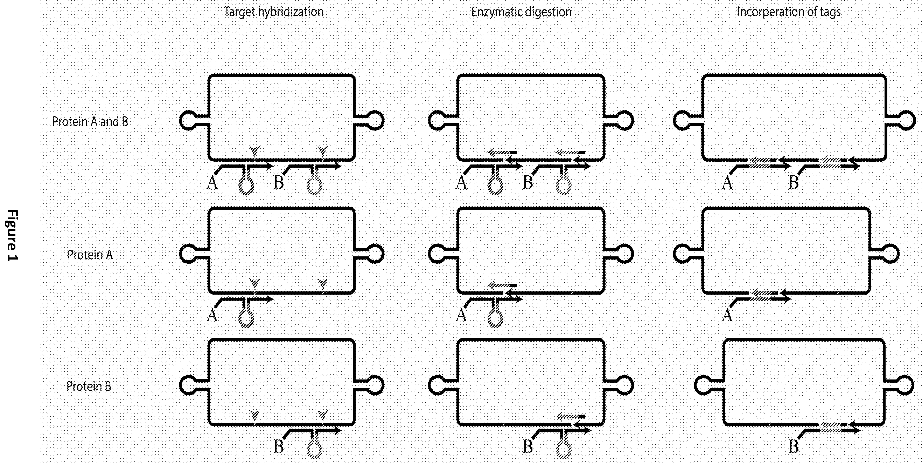

**Figure 1****:** A Schematic presentation of the oligonucleotide designs. The IR circle is hybridized to two (top row) respectively one IC probe (bottom rows), consisting of IC oligonucleotides conjugate to antibodies (indicated as A and B) (left panel). The IR circle is digested by enzyme treatment, where the IR circle hybridizes to an IC probe, and the tag oligonucleotide invades the IC probes (middle panel). The IR circles are religated and the tag sequence gets incorporated (right panel)

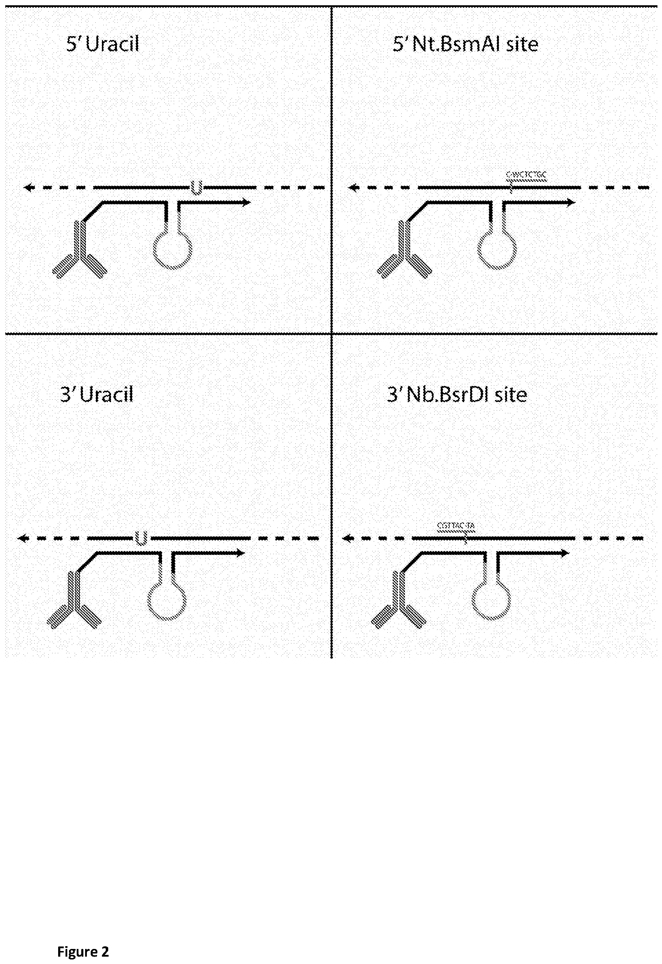

**Figure 2****:** A schematic presentation of the different designs for enzymatic digestion of IR circles, indicating positions of the uracil bases and restriction sites.
**Figure 3****:** Quantification of mean numbers of signals +/- SD from three separate images using the MolBoolean method for detection of β-catenin, E-cadherin and β-catenin-E-cadherin interactions. Images showing cells labeled for β-catenin, E-cadherin and β-catenin-E-cadherin interactions. The borders of the cell nuclei are marked out in the images.

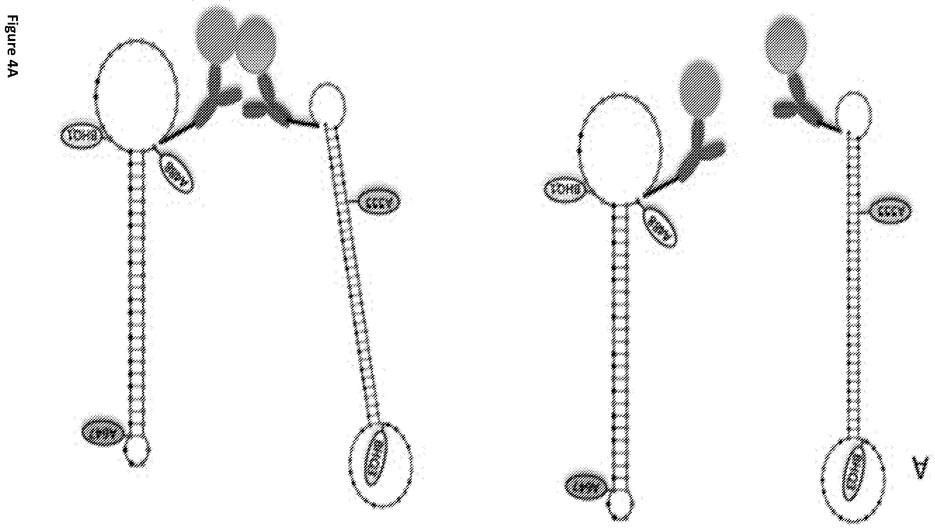

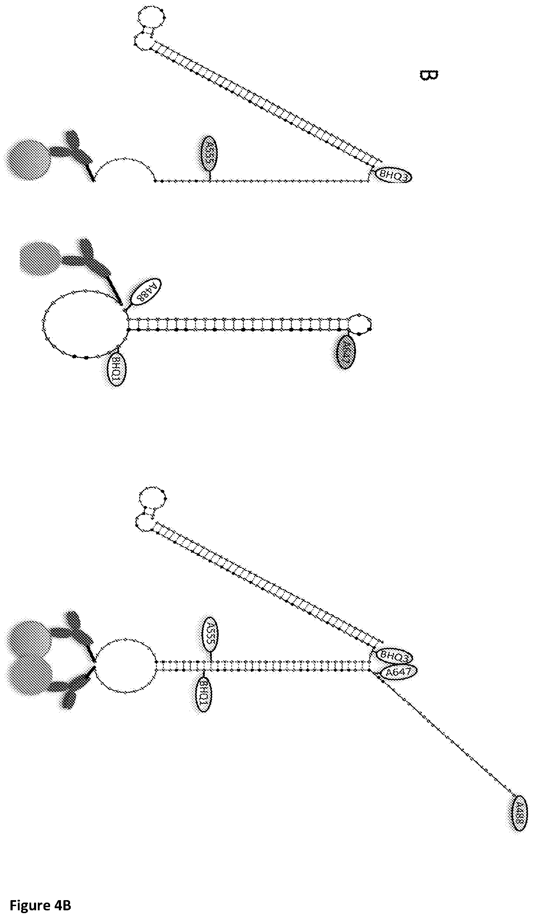

**Figure 4****:** A schematic presentation of a design for Invader-MolBoolean, using three Alexa dyes and two Black hole quenchers. **(A)** When the proximity probes (antibodies conjugated to IC oligonucleotide 1 or 2 (see Table)) bind individual antigens one fluorophore per probe will be able to emit light: Alexa555 (A555) and Alexa647 (A647), while the Alexa488 (A488) will be quenched as it is located close to a quencher (BHQ1). **(B)** When an activator oligonucleotide is added, it will invade IC oligonucleotide 1. If the proximity probes are in close proximity the opened IC oligonucleotide 1 will hybridize to IC oligonucleotide 2 thereby restructuring the positions of fluorophores and quenchers. Now the A555 will be located close to BHQ1 and A647 close to the quencher (BHQ3) - both of these fluorophores will be quenched, while A488 now is separated from the quencher BHQ1 and will emit light.

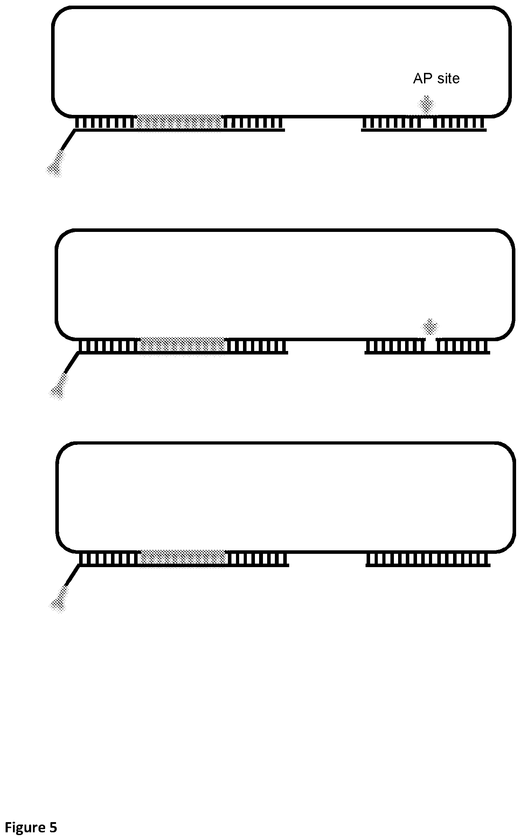

**Figure 5****:** A schematic presentation of the gap-fill process. At first, a digestion template is hybridized over the AP site allowing the circle to be digested by EndolV. Thereafter, the nick created (arrow) can be filled by T4 DNA polymerase, which will incorporate a thymidine. A ligase will seal the nicked circle, so that it can be amplified in the next step of the protocol.

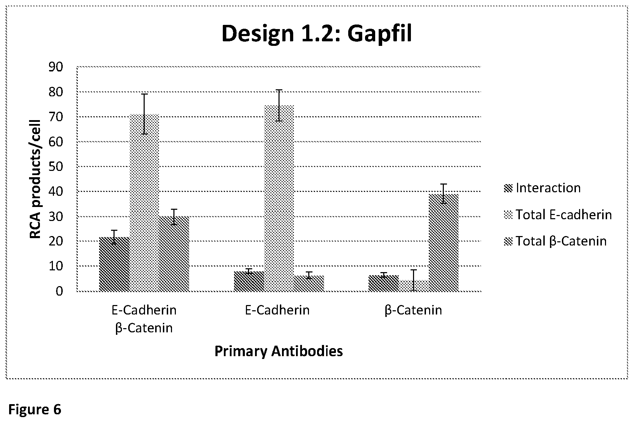

**Figure 6****:** *In situ* protein detection with gap-fill design. The probes were incubated on HaCat cells under four different conditions: with both anti-E-cadherin antibodies and anti-β-catenin antibodies, with anti-E-cadherin antibodies, with anti-β-catenin antibodies or without any primary antibodies (background). All conditions showed were normalized by subtracting the background condition. Error bar represent standard error of the mean (SEM).

### Detailed description of invention

The inventors of the present invention have developed a general method for determining levels of interactions between biomolecules, such as proteins, in a sample, comprising providing a first and a second information carrying (IC) oligonucleotide, wherein the first and second IC oligonucleotide are attached, covalently or non-covalently, to a first and a second affinity reagent, such as antibodies, that have the capacity to bind to a first and a second biomolecule, wherein the first and second IC oligonucleotide, each comprises at least one single-stranded stretch that is complementary to a part of another oligonucleotide, thereby, upon hybridisation of the at least one single-stranded stretch in at least one of the first and second IC oligonucleotides to its complementary part of another oligonucleotide, enabling measurement of the relative proportion of interacting and non-interacting first and second biomolecules in the sample at a single cell or single molecular level.

The method will be exemplified in the present description by two alternative approaches; one that uses fluorophore/quencher technology in combination with hairpin structures in the IC oligonucleotides that are designed so that only one fluorophore per IC oligonucleotide can emit light, and one that, in addition to the IC oligonucleotides, uses an IR (information-receiving) oligonucleotide that carries at least two cleavage motif sites that must become double-stranded in order to allow cleavage. By using any of these approaches measurement of the relative proportion of interacting and non-interacting biomolecules in a sample can be measured at a single cell or single molecular level.

Thus, for one of the aspects of the method of the invention, the inventors of the present invention designed an oligonucleotide system consisting of a single-stranded circular DNA molecule (information receiver (IR)), which carries a motive for cleavage, e.g. uracils or restriction sites. The oligonucleotide system was created so that the cleavage would require the sites to be double-stranded, which it only will be if it binds a complementary oligonucleotide. These complementary oligonucleotides (information carrier (IC)) were designed so that they consist of a hairpin structure flanked with stretches of single-stranded DNA that would be complementary to the IR circle. The nicks created in the IR circles will facilitate a subsequently added short tag oligonucleotide complementary to part of the IC probes to invade the hairpins of the IC oligonucleotides and be ligated into the IR circle. Alternatively, gap filling can be made with a DNA-polymerase, with template from the IC probes. To seal the gaps and recreate a circular DNA molecule, DNA ligase is added. Unique tags and hairpins of the IC oligonucleotides can be used to transfer information to the IR circle of which IC oligonucleotide it has bound. When an IR circle binds two different IC oligonucleotides both corresponding tags will be incorporated. By conjugating the IC oligonucleotides to antibodies, as in one embodiment, the inventors have created a method where the IR circles can be used to monitor if two proteins are interacting, i.e. if both tags are incorporated into the IR circle. The antibodies are chosen against the proteins one intends to study. For the proteins that are not interacting only one tag will be incorporated into the IR circle **(****Figure 1****).**

Different approaches for cutting open the circle and integrating the tag were tested. The IR circle can be cut open by either a nicking endonuclease that only cleaves one of the strands in its double stranded target. Alternatively, a nick could be created by removing a uracil base with the help of the enzyme combination UNG and EndolV. For the nicking endonucleases the inventors tested two different enzymes: Nb.BsrDl, which will make a cut on the 3' side of the hairpin (3' Nb.BsrDI), and Nt.BsmAl, which cuts on the 5' side (5' Nt.BsmAl). The inventors also tested the efficiency in cleavage by incorporating the uracil base in either the 3' side or the 5' side (3'Uracil and 5'Uracil) in relation to the hairpin. **(****Figure 2****).** With the help of Nupack.org, the inventors designed and analyzed all the oligonucleotide sequences to ensure correct secondary structures and hybridizations **(Table 1).**

The recreated IR circles, containing the incorporated tags, can then be amplified by rolling circle amplification (RCA), primed from one of the IC probes. The RCA products contain several hundred complementary repeats of the IR. The identities of the RCA products can then be decoded by hybridization of labeled detection oligonucleotides, complementary to the parts of the RCA product where the tags have been incorporated. The labeling of the detection oligonucleotides could be e.g. different fluorophores, enzymes or molecules with different masses, depending upon which read-out will be used. Single labeled RCA product will be a result of only one incorporated tag while dual labeled RCA products will be a consequence of incorporation of two tags.

An example of how the result can look like is shown in **Figure 3****.** Here the inventors used 5' uracil design, conjugating the IC oligonucleotides to anti-mouse and anti-rabbit antibodies. These IC probes were tested on the MCF10 cell line labeled with a mouse antibody targeting E-cadherin and a rabbit antibody targeting β-Catenin. Complexes containing β-catenin and E-cadherin will result in RCA products detected with both Cy3 and FITC, while the non-interacting proteins gave rise to RCA products labeled with either Cy3 or FITC. The different identities of RCA products were determined using the software CellProfiler and the different types of RCA products were pseudo-colored to visualize interaction between β-catenin and E-cadherin as well as the individual proteins, β-Catenin and E-Cadherin.

An alternative approach to extract the information is to use an IR circle or linear IR molecule to interrogate proximity between IC probes. After ligation of tag sequences the recreated IR molecule can be amplified by PCR and separated by gel electrophoresis. Differences in size will indicate incorporation of different tags. It would also be possible to amplify single IR molecules and decode the identities of different tags by sequencing of the amplicons.

The MolBoolean analyses can be performed in cells, in mixtures of proteins (either in bulk mixtures or separated e.g. by gel electrophoresis). The recreated IR molecules can either be interrogated directly in the sample where they have been formed (e.g. by microscopy, as shown in **Figure 3****)** if the read-out platform supports single molecule detection, or the recreated IR molecules can be collected and single molecules can subsequently be sorted and analyzed individually.

In some instances, remaining abasic sites (AP sites) in the IR molecule can interfere with the amplification and/or detection efficiency. As a means for improving the detection efficiency, and to reduce the risk of undigested abasic sites (AP sites) of the circular IR molecules to prevent detection of signal, the inventors modified the design with an alternative design version **(****Figure 5****).** In order to remove the abasic sites two steps were added after the step where the tags are incorporated. In the first additional step a digestion template was hybridized to the DNA circle, making the area around the AP site double stranded. The abasic site was thereafter removed by digestion by EndolV. In the next step the gap-fill was performed with T4 DNA polymerase to add the missing thymidine and with T4 ligase to close the nick by ligation.

The gap-fill design was evaluated using the previously described E-cadherin and β-catenin interaction assay **(****Figure 6****).** E-cadherin and β-catenin interaction was detected together with a noticeably higher amount of total E-cadherin, which was equal to the total amount E-cadherin when only that antibody was present. Likewise, the total amount of β-catenin remains on a comparable level in both conditions where that antibody was used.

For the other aspect of the method of the invention, the inventors developed an alternative approach to monitor both free and interacting proteins. In this approach, the IC oligonucleotides that are connected to the antibodies are designed to contain hairpin structures. By attaching fluorophores and quenchers to such hairpins the fluorophores will be allowed to emit light only if they are separated in distance from the quenchers. The positioning of fluorophores and quenchers will facilitate that only one fluorophore of each such IC oligonucleotide will be allowed to emit light. Once the IC oligonucleotide has bound their targets, guided by the antibodies connected to them, one of the hairpin structures will be destabilized by invasion of an activator oligonucleotide. This will change the conformation of the IC oligonucleotide and will reveal a stretch of DNA that previously was hidden in the stem, which is reverse complementary to a stretch of the second IC oligonucleotide. The destabilized/activated first IC oligonucleotide will hybridize to the second IC oligonucleotide, only if they are bound to target molecules in close proximity, which will reposition the fluorophores and quenchers so that the ones that previously emitted light now are quenched and one fluorophores that was quenched in one of the IC oligonucleotide gets separated from the quencher. This fluorophore will only be able to emit light if a pair of IC oligonucleotides is hybridized to each other. Hence, the fluorescence of free and interacting proteins can be recorded simultaneously at different wavelengths. This method, herein called Invader-MolBoolean is described in **Figure 4****.** With the help of Nupack.org, the inventors designed and analyzed all the oligonucleotide sequences to ensure correct secondary structures and hybridizations **(Table 1).**

The invention will now be described with reference to the following non-limiting examples.

### Examples

### Example 1- In situ detection of Beta-catenin and E-cadherin interaction:

The information receiving DNA circle was created by ligating two single stranded pieces (i.e. IR circle piece 1 and 2 (see Table1)), which carries motifs that will ensure proper hybridization, i.e. the two hairpin structures in the circle. The two oligonucleotides were mixed together at a final concentration of 1 µM in T4 Ligation buffer. Thereafter 0.02 U/µl of T4 ligase was added in the mix and it was incubated for 2 h at 37°C followed by 48 h incubation at 4°C.

350 µg antibody, donkey-anti-rabbit or donkey-anti-mouse (Jackson ImmunoResearch, West Grove, USA) per conjugated PLA probe, were concentrated using the Amicon Ultra 10K centrifugal filter unit (Merck Millipore, Massachusetts, USA, according to manufacturer's instructions to the concentration 3 mg/ml in PBS. S-HyNic Crosslinker (Solulink, San Diego, USA) was dissolved in DMSO to 20 mM and the crosslinker and antibody was mixed with a 25x molar excess of crosslinker over antibody. The mix was incubated with gentle agitation at room temperature, and protected from light, for 2 hours. After activation of the antibodies the buffer was exchanged to 100 mM NaHPO4, 150 mM NaCl, pH 6.0 buffer by prewashed Zeba Spin Desalting Columns 7K MWCO (Thermo Scientific). Subsequently to the buffer exchange the antibody was mixed with an aldehyde modified oligonucleotide **(Table 1)** at an antibody:oligonucleotide ratio of 1:3. Aniline was added, at a final concentration of 10 mM, to catalyze the reaction. The antibody oligonucleotide mix was incubated with gentle agitation at room temperature, and protected from light, for 2 hours. Immediately after the incubation the buffer were exchanged to PBS using prewashed Zeba Spin Desalting Columns 7K MWCO (Life Technologies). After conjugation the conjugates were purified from unconjugated antibody and oligonucleotide by ÄKTA Pure HPLC (GE Healthcare, Uppsala, Sweden) using Superdex 200 10/300 column (GE Healthcare). The collected fractions from the HPLC purification were concentrated to 80 µl by Amicon Ultra 10K centrifugal filter unit (Merck Millipore) according to manufacturer's instructions and validated by electrophoresis. The conjugates were mixed with Novex TBE-Urea Sample buffer (Life Technologies) and separated on Novex TBE-Urea Gel 10% (Life Technologies) by 180 V for 50 minutes. DNA was visualized using SYBR Gold Nucleic Acid Gel Stain (Life Technologies) and protein using Coomassie stain (Bio-Rad, Hercules, USA). The gel was visualized with Bio-Rad Gel-Doc XR (Bio-Rad). The concentrations of the conjugates were determined using the Pierce BCA protein assay kit (Life Technologies).

Cells on a microscope slide was fixated by 3.7% PFA for 10 minutes and then permeabilized in 1x PBS with 0.2% triton X100, and thereafter washed twice in 1x PBS before adding blocking solution; 50% Odyssey blocking buffer (cat. #927-50000, LiCor) in 1x TBS. After 30 min of blocking at 37°C in a moister chamber, the cells were incubated with anti E-cadherin (cat. # BD610182, BD biosciences, diluted 1:100) and anti- Beta-catenin (cat. # sc7199, santacruz, diluted 1:200) in blocking solution overnight at 4°C. Each slide was washed three times for three minutes in 1xTBS with 0.05% Tween 20 (TBST).

The Molboolean IC probes were mixed in blocking solution, at a final concentration of 100 ng/ml, and incubated with the cells for 60 minutes at 37 °C. The slides were then washed three times, for three minutes, in TBST. The Molboolean IR circle was diluted to 0.025 µM in T4 ligation buffer supplemented with 0.25 mg/ml of BSA to allow for hybridization of the circle to the IC probes while incubating it for 30 minutes at 37°C with the cells. The cells are thereafter washed twice in TBST for three minutes each and are then incubated with digestion enzymes dependent on the design at 37°C. The 5'Uracil design is digested by 0.1 U/µl of Endo IV and 0.05 U/µl UNG in 20 mM Tris-HCI (pH 7,6) buffer with 30 mM NaCl, 1mM EDTA, 100mM KCI, 1mM DTT and 0.25 mg/ml of BSA fo 45 min before washing. While, the 5' Nt.BsmAI design is incubated with 0.125 U/µl Nt.BsmAI enzyme diluted in 1x NEBuffer Cut smart supplemented with 0.25 mg/ml BSA for 1 h. The cells were washed for 3 min twice in TBST and were thereafter incubated for 30 min at 37°C with Tag 1 and 2 both at 0.125 µM in the presence of 0.05 U/µL T4 Ligase in T4 ligation buffer supplemented with 0.25mg/ml BSA. After ligation, there is another wash in TBST (2x3min).

The gap-fill design introduces two extra steps. The slides were incubated with 0.05 µM of digestion template together with 0.01 U/µl of Endo IV in 20 mM Tris-HCI (pH 7,6) buffer with 30 mM NaCl, 1 mM EDTA, 100 mM KCI, 1 mM DTT and 0.25 mg/ml of BSA for 60 min at RT. The slides were washed three times for 3 min in TBST. To fill the gap, the slides were incubated with 0.025 U/µl T4 DNA polymerase and 0.05 U/µl T4 ligase in 1xT4 ligase buffer supplemented with 0.25mg/ml BSA and 0.1 mM dNTP for 30 min at RT. The cells were thereafter washed twice in TBST for 3 min each.

Thereafter, a RCA reaction is performed on the newly formed circles to amplify the signal for 60 min at 37°C. The RCA is catalyzed by 0.5 U/µl phi29 in the following solution: 33 mM Tris-, 10 mM Mg-acetate, 66 mM K-acetate, 0.1% Tween 20, 1 mM DTT, 7.5 ng/ml PolyA, and 0.25 mM dNTP. It was washed twice for three minutes in TBST. The RCA products were detected by hybridizing 0.025 µM DO1 and 2 to it in PBS supplemented with 0.0025 µg/ml salmon sperm DNA and 0.25mg/ml BSA, and the mixture also contained 0.04 mg/ml Hoechst 33342 for nuclei staining.

The staining was followed by two 10 min washes in 1xTBS and a 15 min wash in 0.2x TBS and the slide was thereafter dried with 70% etOH and mounted using Vectasheld.

**Table 1**

| Design | Description | SEQ ID NO | Sequence |
|---|---|---|---|
| Universal | IR circle piece 1 | | |
| 5' Uracil | IR circle piece 2 | 2 | |
| | Tag 1 | 3 | P-TCTGCAGTTATACGTCCAATCATAA |
| | Tag 2 | 4 | P-TCGTACGTAGATCCTGCCATTTCTA |
| | IC oligo 1 | 5 | |
| | IC oligo 2 | 6 | |
| | Gap fill 1 | 7 | TAGGTAGTGGGCAGAGGTAGGAGTGGACA |
| | Gap fill 2 | 8 | AGGTGGAATAGACGAGGCAGGTGAAGTAG |
| Nt.BsmAl | IR Circle piece 2 | 9 | |
| | Tag 1 | 10 | P-CTGCGCAGTTATACGTCCAATCATAA |
| | Tag 2 | 11 | P-CGTACGTAGATCCTGCCATTTCTA |
| | IC oligo 1 | 12 | |
| | IC oligo 2 | 13 | |
| | | | |
| 5' Uracil and Nt.BsmAl | Detection Oligo1 - Cy3 | 14 | Cy3-TCTGCAGTTATACGTCCAATUUU |
| | Detection Oligo2 - FITC | 15 | FITC-TCGTACGTAGATCCTGCCATUUU |
| 3' Uracil | IR circle piece 2 | 16 | |
| | Tag 1 | 17 | P-TCACGTCCAATCGATAACTACAGTTAT |
| | Tag 2 | 18 | P-TTCCTGCCATTATCTACGTGTAGAT |
| | IC oligo 1 | 19 | |
| | IC oligo 2 | 20 | |
| Nb.BsrDl | IR circle piece 2 | 21 | |
| | Tag 1 | 22 | P-CACGTCCAATCGATAACTACAGTTAT |
| | Tag 2 | 23 | P-TCCTGCCATTATCTACGTGTAGAT |
| | IC oligo 1 | 24 | |
| | IC oligo 2 | 25 | |
| 3' Uracil and Nb.BsrDl | Detection Oligo1 - Cy3 | 26 | Cy3-TCCAATCGATAACTACAGTTAT |
| | Detection Oligo2 - FITC | 27 | FITC-TGCCATTATCTACGTGTAGAT |
| Invader-Molboolean | IC oligonucleotide1 | 28 | |
| | IC oligonucleotide2 activator | 29 | |
| | | 30 | CCTACTTCCACTCCCATCACTTACCTACTTCACTTCACC |

## Claims

1. Method for determining levels of interactions between biomolecules, such as proteins, in a sample, comprising providing a first and a second information carrying (IC) oligonucleotide, wherein the first and second IC oligonucleotide are attached, covalently or non-covalently, to a first and a second affinity reagent, such as antibodies, that have the capacity to bind to a first and a second biomolecule, wherein the first and second IC oligonucleotide each comprises at least one single-stranded stretch that is complementary to a part of either an information receiving (IR) oligonucleotide or an activating oligonucleotide, thereby, upon hybridisation of the at least one single-stranded stretch in at least one of the first and second IC oligonucleotides to its complementary part of another oligonucleotide, enables measurement of the relative proportion of interacting and non-interacting first and second biomolecules in the sample at a single cell or single molecular level, wherein the single-stranded stretch of the first and second IC oligonucleotides enables interaction
(a) between the first and/or second IC oligonucleotide and an information receiving (IR) oligonucleotide, wherein the (IR) oligonucleotide carries at least two cleavage motif sites that upon hybridization to an IC oligonucleotide become double-stranded to allow cleavage, or
(b) between the first and/or second IC oligonucleotide and an activating oligonucleotide, wherein the IC oligonucleotides connected to said antibodies are designed to contain hairpin structures, and wherein said IC oligonucleotides are modified with fluorophores and quenchers, which are positioned so that only one fluorophore per oligonucleotide can emit light, wherein the hybridization between the first and/or second IC oligonucleotide and the activating oligonucleotide restructures the positions of fluorophores and quenchers, such that the one signalling fluorophore allows for discrimination between interacting and non-interacting biomolecules.

2. Method according to claim 1, comprising the steps of:
a. providing the single-stranded information receiving (IR) DNA molecule, wherein the IR DNA molecule is circular or linear, carrying at least a first and a second cleavage motif, wherein the cleavage motifs are chosen so that the cleavage motif sites must become double-stranded in order to allow cleavage;
b. providing the first information carrying (IC) DNA molecule, comprising a single-stranded stretch that is complementary to the part of the IR DNA molecule carrying the first cleavage motif, wherein the occurrence of the first IC DNA molecule reflects the amount of a first biomolecule in the sample;
c. providing the second information carrying (IC) DNA molecule, comprising a single-stranded stretch that is complementary to the part of the IR DNA molecule carrying the second cleavage motif, wherein the occurrence of the second IC DNA molecule reflects the amount of a second biomolecule in the sample
d. mixing the DNA molecules of step a-c under conditions that allow binding of complementary single-stranded stretches;
e. adding digestion enzyme(s) to create nick(s) at the cleavage motif site(s) that has/have become double-stranded, thereby forming
i. a first reporter tag binding site that will allow binding of a first reporter tag DNA molecule or sequence, comprising a single-stranded stretch that is complementary to a part of the first IC DNA molecule, and/or
ii. a second reporter tag binding site that will allow binding of a second reporter tag DNA molecule or sequence comprising a single-stranded stretch that that is complementary to a part of the second IC DNA molecule;
f. incorporating reporter tag DNA sequences by any one of the following alternatives:
i. adding the first reporter tag DNA molecule and the second reporter tag DNA molecule, whereby the first reporter tag DNA molecule is incorporated in the IR DNA molecule at the first reporter tag binding site if a nick has been created at the first cleavage motif site, and/or the second reporter tag DNA molecule is incorporated in the IR DNA molecule at the second reporter tag binding site if a nick has been created at the second cleavage motif site; or
ii. using a DNA polymerase and nucleotides to incorporate the first reporter tag DNA sequence in the IR DNA molecule by filling the gap complementary to the first reporter tag binding site on the first IC oligonucleotide if a nick has been created at the first cleavage motif site, and/or to incorporate the second reporter tag DNA sequence in the IR DNA molecule by filling the gap complementary to the second reporter tag binding site on the second IC oligonucleotide if a nick has been created at the second cleavage motif site; and adding a ligation enzyme ligating the IR DNA molecule, thereby providing a recreated IR DNA molecule;
g. optionally amplifying the recreated IR DNA molecule of step f;
h. monitoring the incorporation of the first and/or second reporter tag(s) in the recreated IR DNA molecule, as a measurement of the occurrence of and/or interaction between the first and/or the second biomolecule(s) in the sample.

3. Method according to claim 2, wherein cleavage motifs are chosen from uracils or restriction sites.

4. Method according to claim 2 or 3, wherein the digestion enzyme(s) used to create nicks at the cleavage motif sites is/are chosen from
i. nicking endonuclease 3' Nb.Bsr.DI nicking 3' of a double stranded restriction site,
ii. nicking endonuclease 5' Nt.BsmAI nicking 5' of a double stranded restriction site, or
iii. a combination of Uracil-DNA glycolsylase (UDG), removing a uracil base at a double stranded site, and EndolV, removing the apyrimidinic site.

5. Method according to any one of claims 2-4, wherein the monitoring is performed by
a. providing a first labeled detection oligonucleotide that is complementary to at least a part of the first reporter tag DNA molecule, and a second labeled detection oligonucleotide that is complementary to at least a part of the second reporter tag DNA molecule, and
b. hybridizing the first and second labeled detection oligonucleotides to the recreated IR DNA molecule, which optionally is amplified.

6. Method according to any one of claims 2-5, wherein a linear recreated IR DNA molecule is amplified and thereafter separated by a separation method, such as electrophoresis or chromatography, wherein separation products having different sizes indicate incorporation of different reporter tags.

7. Method according to any one of claims 2-6, wherein the identities of different reporter tags are monitored by sequencing.

8. Method according to any one of claims 2-7, wherein the recreated IR DNA molecule is monitored in the sample where it has been formed, such as by microscopy, or wherein the recreated IR DNA molecule is collected from the sample where it has been formed followed by sorting and analysis of single molecules, such as by microscopy.

9. Method according to any one of claims 2-8, for removing abasic sites and improving detection efficiency, wherein after the reporter tag molecules have been added and the first and/or second reporter tag have/has been incorporated into the IR, the following steps are performed:
i. hybridizing a digestion template to the IR DNA molecule making the area around the remaining abasic site double stranded;
ii. removing the abasic site by digesting the double stranded area by a suitable restriction enzyme, such as EndolV;
iii. gap-filling the digested area with a suitable polymerase, such as T4 DNA polymerase, to add the missing base, such as thymidine; and ligating the IR DNA molecule with a ligase, such as T4 ligase, to provide a recreated IR DNA molecule.

10. Method according to claim 1,
a. wherein the first and second IC oligonucleotides comprise hairpin structures in which fluorophores and quenchers are positioned, wherein the hairpin structures are designed so that only one fluorophore per oligonucleotide can emit light, and wherein each fluorophore has a unique signal;
b. wherein the hairpins in the first and second IC oligonucleotides are disrupted or destabilized by providing an activating oligonucleotide that is complementary to the first or the second IC oligonucleotide thereby binding to the first or second IC oligonucleotide so that the first and second IC oligonucleotides can interact with each other causing a repositioning of fluorophores and quenchers;
c. wherein pairs of conjugates, comprising affinity reagents coupled to the first or second IC oligonucleotide, are used to interrogate proximity between two biomolecules to which the affinity reagents bind, wherein a first fluorophore signal pattern will be exhibited upon interaction between the first and second biomolecule, and a second fluorophore signal pattern will be exhibited upon lack of interaction between the first and second biomolecule, as a result of the oligonucleotides hybridizing to each other upon interaction between the first and second biomolecule causing restructuring of the positions of fluorophores and quenchers.

11. Method according to any one of the preceding claims, wherein the first and the second biomolecules are proteins or polypeptides.

12. Method according to any one of the preceding claims, wherein the sample is a biological sample of one or more cells, or a mixture of proteins.

13. Method according to any one of the preceding claims, wherein the first IC DNA molecule is conjugated to a first antibody molecule being equal to or targeting the first biomolecule, and the second IC DNA molecule is conjugated to the second antibody molecule being equal to or targeting the second biomolecule.

14. Method according to any one of the preceding claims for analysing protein-protein interactions in single cells or single molecules.

15. Kit for use in the method of any one of claims 1-9, 11-14, comprising
a. a single-stranded information receiving (IR) DNA molecule, carrying a first and a second cleavage motif;
b. a first and a second information carrying (IC) DNA molecule, reflecting the amounts of a first and a second biomolecule in a sample, wherein the first and second IC DNA molecules are conjugated to affinity reagents or provided with chemical moieties to be conjugated by a kit user;
c. optionally enzymes for creating nicks at the cleavage motif sites in the IR DNA molecule;
d. a first and a second reporter tag DNA molecule;
e. optionally reagents for amplification of a recreated IR DNA molecule;
f. optionally a first and a second labelled detection oligonucleotide; and g. optionally DNA molecule(s) and enzymes to remove abasic sites

16. Kit for use in the method of any one of claims 1, 10-14, comprising
a. a first and a second information carrying (IC) DNA molecule comprising hairpin structures in which fluorophores and quenchers are positioned, wherein the hairpin structures are designed so that only one fluorophore per oligonucleotide can emit light, and wherein each fluorophore has a unique signal, thereby having the ability to reflect the amounts of a first and a second biomolecule in a sample, wherein the first and second IC DNA molecules are conjugated to affinity reagent or are provided with chemical moieties to be conjugated by a kit user; and
b. an activating oligonucleotide that is complementary to the first or the second IC DNA molecule thereby having the ability to bind to the first or second IC DNA molecule.

## Patentansprüche

1. Verfahren zur Bestimmung des Ausmaßes an Wechselwirkungen zwischen Biomolekülen, wie etwa Proteinen, in einer Probe, wobei im Rahmen desselben ein erstes und ein zweites informationstragendes (IC) Oligonukleotid bereitgestellt werden, wobei die ersten und zweiten IC-Oligonucleotide, auf kovalente oder nicht-kovalente Weise, an einem ersten und einem zweiten Affinitätsreagenz, wie etwa Antikörpern, befestigt sind, welche dazu befähigt sind, an ein erstes und ein zweites Biomolekül zu binden, wobei das erste und das zweite IC-Oligonukleotid jeweils mindestens einen einzelsträngigen Abschnitt umfassen, welcher komplementär zu einem Teilbereich entweder eines informationsempfangenden (IR) Oligonukleotids oder eines aktivierenden Oligonukleotids ist, sodass eine Hybridisierung des mindestens einen einzelsträngigen Abschnitts in mindestens einem der ersten und zweiten IC-Oligonukleotide mit seinem komplementären Teilbereich eines anderen Oligonukleotids es ermöglicht, den relativen Anteil an wechselwirkenden und nicht-wechselwirkenden ersten und zweiten Biomolekülen in der Probe auf der Ebene einer einzigen Zelle oder eines einzigen Moleküls zu messen, wobei der einzelsträngige Abschnitt der ersten und zweiten IC-Oligonukleotide Wechselwirkungen der folgenden Art ermöglicht
(a) zwischen dem ersten und/oder zweiten IC-Oligonukleotid und einem informationsempfangenden (IR) Oligonukleotid, wobei das (IR)-Oligonukleotid mit mindestens zwei Spaltungsbaustein-Stellen versehen ist, die bei der Hybridisierung an ein IC-Oligonukleotid doppelsträngig werden, um eine Spaltung zu ermöglichen, oder
(b) zwischen dem ersten und/oder zweiten IC-Oligonukleotid und einem aktivierenden Oligonukleotid, wobei die IC-Oligonukleotide, welche an die Antikörper gebunden sind, derart ausgelegt sind, dass sie Haarnadelstrukturen enthalten, und wobei die IC-Oligonukleotide mit Fluorophoren und Quenchern modifiziert sind, welche derart angeordnet sind, dass pro Molekül nur ein Fluorophore Licht aussenden kann, wobei die Hybridisierung zwischen dem ersten und/oder zweiten IC-Oligonukleotid und dem aktivierenden Oligonukleotid eine derartige Umstrukturierung der Positionen von Fluorophoren und Quenchern bewirkt, dass das eine signalgebende Fluorophor eine Unterscheidung zwischen wechselwirkenden und nicht-wechselwirkenden Biomolekülen ermöglicht.

2. Verfahren gemäß Anspruch 1, welches die folgenden Schritte umfasst:
a. Bereitstellen des einzelsträngigen informationsempfangenden (IR) DNA-Moleküls, wobei das IR-DNA-Molekül kreisförmig oder geradkettig ist, mit mindestens einem ersten und einem zweiten Spaltungsbaustein versehen ist, wobei die Spaltungsbausteine derart ausgewählt sind, dass die Spaltungsbaustein-Stellen doppelsträngig werden müssen, um eine Spaltung zu ermöglichen;
b. Bereitstellen des ersten informationstragenden (IC) DNA-Moleküls, welches einen einzelsträngigen Abschnitt umfasst, der komplementär zu dem Teilbereich des IR-DNA-Moleküls ist, welches mit dem ersten Spaltungsbaustein versehen ist, wobei die Häufigkeit des ersten IC-DNA-Moleküls die Menge eines ersten Biomoleküls in der Probe widerspiegelt;
c. Bereitstellen des zweiten informationstragenden (IC)-DNA-Moleküls, welches einen einzelsträngigen Abschnitt umfasst, der komplementär zu dem Teilbereich des IR-DNA-Moleküls ist, welches mit dem zweiten Spaltungsbaustein versehen ist, wobei die Häufigkeit des zweiten IC-DNA-Moleküls die Menge eines zweiten Biomoleküls in der Probe widerspiegelt
d. Mischen der DNA-Moleküle der Schritte a bis c unter Bedingungen, die eine Bindung komplementärer einzelsträngiger Abschnitte ermöglichen;
e. Zusetzen eines/von verdauend wirkenden Enzyms/Enzymen, um (einen) Einzelstrangbruch/Einzelstrangbrüche an der/den Spaltungsbaustein-Stelle(n) zu erzeugen, welche doppelsträngig geworden ist/sind, um auf diese Weise Folgendes zu bilden
i. eine erste Reporter-tag-bindende Stelle, die eine Bindung eines ersten Reporter-tag-DNA-Moleküls oder einer solchen Sequenz ermöglichen wird, welche(s) einen einzelsträngigen Abschnitt umfasst, der komplementär zu einem Teilbereich des ersten IC-DNA-Moleküls ist, und/oder
ii. eine zweite Reporter-tag-bindende Stelle, die eine Bindung eines zweiten Reporter-tag-DNA-Moleküls oder einer solchen Sequenz ermöglichen wird, welche(s) einen einzelsträngigen Abschnitt umfasst, der komplementär zu einem Teilbereich des zweiten IC-DNA-Moleküls ist;
f. Einfügen von Reporter-tag-DNA-Sequenzen mittels einer der folgenden Alternativen:
i. Hinzufügen des ersten Reporter-tag-DNA-Moleküls und des zweiten Reporter-tag-DNA-Moleküls, wobei das erste Reporter-tag-DNA-Molekül an der ersten Reporter-tag-bindenden Stelle in das IR-DNA-Molekül eingefügt wird, wenn an der ersten Spaltungsbaustein-Stelle ein Einzelstrangbruch erzeugt wurde, und/oder das zweite Reporter-tag-DNA-Molekül an der zweiten Reporter-tag-bindenden Stelle in das IR-DNA-Molekül eingefügt wird, wenn an der zweiten Spaltungsbaustein-Stelle ein Einzelstrangbruch erzeugt wurde; oder
ii. Verwenden einer DNA-Polymerase und von Nukleotiden, um die erste Reporter-tag-DNA-Sequenz in das IR-DNA-Molekül einzufügen, indem die Lücke, welche komplementär zu der ersten Reporter-tag-bindenden Stelle auf dem ersten IC-Oligonukleotid ist, gefüllt wird, wenn an der ersten Spaltungsbaustein-Stelle ein Einzelstrangbruch erzeugt wurde, und/oder um die zweite Reporter-tag-DNA-Sequenz in das IR-DNA-Molekül einzufügen, indem die Lücke, welche komplementär zu der zweiten Reporter-tag-bindenden Stelle auf dem zweiten IC-Oligonukleotid ist, gefüllt wird, wenn an der zweiten Spaltungsbaustein-Stelle ein Einzelstrangbruch erzeugt wurde; und Zusetzen eines Ligationsenzyms, welches das IR-DNA-Molekül ligiert, um auf diese Weise ein wiederhergestelltes IR-DNA-Molekül bereitzustellen;
g. möglicherweise Vervielfältigen des wiederhergestellten IR-DNA-Moleküls des Schrittes f;
h. Überwachen des Einfügens des/der ersten und/oder zweiten Reporter-tag-Element(s)/Elemente in das wiederhergestellte IR-DNA-Molekül, anhand einer Messung der Häufigkeit des ersten und/oder zweiten Biomoleküls/Biomoleküle in der Probe und/oder der Wechselwirkung zwischen diesen.

3. Verfahren gemäß Anspruch 2, wobei die Spaltungsbausteine aus Uracil-Einheiten oder Restriktionsstellen ausgewählt sind.

4. Verfahren gemäß Anspruch 2 oder 3, wobei das/die verdauend wirkende(n) Enzyme(e), welche(s) dazu verwendet wird/werden, Einzelstrangbrüche an den Spaltungsbaustein-Stellen zu erzeugen, aus Folgendem ausgewählt ist/sind
i. Nicking-Endonuklease 3'-Nb.Bsr.DI, welche einen 3'-seitigen Einzelstrangbruch bei einer doppelsträngigen Restriktionsstelle bewirkt,
ii. Nicking-Endonuklease 5'-Nb.BsmAI, welche einen 5'-seitigen Einzelstrangbruch bei einer doppelsträngigen Restriktionsstelle bewirkt, oder
iii. einer Kombination aus Uracil-DNA-Glycosylase (UDG), welche eine Uracil-Base an einer doppelsträngigen Stelle entfernt, und EndolV, welche die apyrimidinische Stelle entfernt.

5. Verfahren gemäß einem beliebigen der Ansprüche 2 bis 4, wobei das Überwachen erfolgt, indem
a. ein erstes markiertes Detektionsoligonukleotid, das komplementär zu mindestens einem Teilbereich des ersten Reporter-tag-DNA-Moleküls ist, und ein zweites markiertes Detektionsoligonukleotid, das komplementär zu mindestens einem Teilbereich des zweiten Reporter-tag-DNA-Moleküls ist, bereitgestellt werden, und
b. die ersten und zweiten markierten Detektionsoligonukleotide mit dem wiederhergestellten IR-DNA-Molekül hybridisiert werden, welches möglicherweise vervielfältigt wird.

6. Verfahren gemäß einem beliebigen der Ansprüche 2 bis 5, wobei ein geradkettiges wiederhergestelltes IR-DNA-Molekül vervielfältigt und danach mittels eines Trennverfahrens wie etwa Elektrophorese oder Chromatographie aufgetrennt wird, wobei Auftrennungsprodukte, welche verschiedene Größen haben, das Einfügen verschiedenartiger Reporter-tags anzeigen.

7. Verfahren gemäß einem beliebigen der Ansprüche 2 bis 6, wobei die Identitäten verschiedenartiger Reporter-tags durch Sequenzierung überwacht werden.

8. Verfahren gemäß einem beliebigen der Ansprüche 2 bis 7, wobei das wiederhergestellte IR-DNA-Molekül in der Probe überwacht wird, in welcher es gebildet wurde, wie etwa mittels Mikroskopie, oder wobei das wiederhergestellte IR-DNA-Molekül ausgehend von der Probe gewonnen wird, in welcher es gebildet wurde, woraufhin die einzelnen Moleküle klassiert und untersucht werden, wie etwa mittels Mikroskopie.

9. Verfahren gemäß einem beliebigen der Ansprüche 2 bis 8, zum Entfernen abasischer Stellen und Verbessern des Detektionswirkungsgrades, wobei nach dem Zusetzen der Reporter-tag-Moleküle und dem Einfügen des/der ersten und/oder zweiten Reporter-tags in das IR die folgenden Schritte durchgeführt werden:
i. Hybridisieren einer Verdau-Vorlage an das IR-DNA-Molekül, sodass der Bereich rundum die verbleibende abasische Stelle doppelsträngig wird;
ii. Entfernen der abasischen Stelle, indem der doppelsträngige Bereich mittels eines zweckmäßigen Restriktionsenzyms, wie etwa EndolV, verdaut wird;
iii. Füllen der Lücke des verdauten Bereichs mit einer zweckmäßigen Polymerase, wie etwa T4-DNA-Polymerase, um die fehlende Base, wie etwa Thymidin, hinzuzufügen; und Ligieren des IR-DNA-Moleküls mit einer Ligase, wie etwa der T4-Ligase, um ein wiederhergestelltes IR-DNA-Molekül bereitzustellen.

10. Verfahren gemäß Anspruch 1,
a. wobei die ersten und zweiten IC-Oligonukleotide Haarnadelstrukturen umfassen, in welchen Fluorophore und Quencher angeordnet sind, wobei die Haarnadelstrukturen derart ausgelegt sind, dass pro Oligonukleotid nur ein Fluorophor Licht aussenden kann, und wobei jedes Fluorophor ein einzigartiges Signal hat;
b. wobei die Haarnadeln in den ersten und zweiten IC-Oligonukleotiden aufgebrochen oder destabilisiert werden, indem ein aktivierendes Oligonukleotid bereitgestellt wird, das komplementär zum ersten oder zum zweiten IC-Oligonukleotid ist, um auf diese Weise derart an das erste oder das zweite IC-Oligonukleotid zu binden, dass die ersten und zweiten IC-Oligonukleotide miteinander in Wechselwirkung treten können, wodurch ein Neuanordnung von Fluorophoren und Quenchern bewirkt wird;
c. wobei Paare von Konjugaten, die Affinitätsreagenzien umfassen, welche an das erste oder zweite IC-Oligonukleotid gekuppelt sind, dazu verwendet werden, die Nähe zwischen zwei Biomolekülen abzufragen, an welche die Affinitätsreagenzien binden, wobei sich ein erstes Muster von Fluorophorensignalen zeigen wird, wenn es zu einer Wechselwirkung zwischen dem ersten und zweiten Biomolekül kommt, und sich ein zweites Muster von Fluorophorensignalen zeigen wird, wenn es an Wechselwirkungen zwischen dem ersten und zweiten Biomolekül fehlt, wobei dies darauf zurückzuführen ist, dass die Oligonukleotide infolge einer Wechselwirkung zwischen dem ersten und zweiten Biomolekül, die eine Umstrukturierung der Positionen von Fluorophoren und Quenchern bewirkt, miteinander hybridisieren.

11. Verfahren gemäß einem beliebigen der vorhergehenden Ansprüche, wobei es sich bei dem ersten und dem zweiten Biomolekül um Proteine oder Polypeptide handelt.

12. Verfahren gemäß einem beliebigen der vorhergehenden Ansprüche, wobei es sich bei der Probe um eine biologische Probe von einer oder mehreren Zellen oder um eine Mischung von Proteinen handelt.

13. Verfahren gemäß einem beliebigen der vorhergehenden Ansprüche, wobei das erste IC-DNA-Molekül mit einem ersten Antikörpermolekül konjugiert ist, welches dem ersten Biomolekül entspricht oder gegen dieses gerichtet ist, und das zweite IC-DNA-Molekül mit dem zweiten Antikörpermolekül konjugiert ist, welches dem zweiten Biomolekül entspricht oder gegen dieses gerichtet ist.

14. Verfahren gemäß einem beliebigen der vorhergehenden Ansprüche, um Protein-Protein-Wechselwirkungen bei einzelnen Zellen oder einzelnen Molekülen zu untersuchen.

15. Kit zur Verwendung in dem Verfahren nach einem beliebigen der Ansprüche bis 9, 11 bis 14, umfassend
a. ein einzelsträngiges informationsempfangendes (IR) DNA-Molekül, das mit einem ersten und einem zweiten Spaltungsbaustein versehen ist;
b. ein erstes und ein zweites informationstragendes (IC) DNA-Molekül, welches die Mengen an einem ersten und einem zweiten Biomolekül in einer Probe widerspiegelt, wobei die ersten und zweiten IC-DNA-Moleküle mit Affinitätsreagenzien konjugiert sind oder mit chemischen Gruppen versehen sind, die vom Anwender des Kits zu konjugieren sind;
c. möglicherweise Enzyme zum Erzeugen von Einzelstrangbrüchen an den Spaltungsbaustein-Stellen in dem IR-DNA-Molekül;
d. ein erstes und ein zweites Reporter-tag-DNA-Molekül;
e. möglicherweise Reagenzien für eine Vervielfältigung eines wiederhergestellten IR-DNA-Moleküls;
f. möglicherweise ein erstes und ein zweites markiertes Detektionsoligonukleotid; und g. möglicherweise (ein) DNA-Molekül(e) und Enzyme zum Entfernen abasischer Stellen.

16. Kit zur Verwendung in dem Verfahren nach einem beliebigen der Ansprüche 1, 10 bis 14, umfassend
a. ein erstes und ein zweites informationstragendes (IC) DNA-Molekül, das Haarnadelstrukturen umfasst, in welchen Fluorophore und Quencher angeordnet sind, wobei die Haarnadelstrukturen derart ausgelegt sind, dass pro Oligonukleotid nur ein Fluorophor Licht aussenden kann, und wobei jedes Fluorophor ein einzigartiges Signal hat, wobei es auf diese Weise dazu befähigt ist, die Mengen eines ersten und eines zweiten Biomoleküls in einer Probe widerzuspiegeln, wobei die ersten und zweiten IC-DNA-Moleküle mit einem Affinitätsreagenz konjugiert sind oder mit chemischen Gruppen versehen sind, welche von einem Anwender des Kits zu konjugieren sind; und
b. ein aktivierendes Oligonukleotid, das komplementär zu dem ersten oder dem zweiten IC-DNA-Molekül ist, sodass es dazu befähigt ist, an das erste oder zweite IC-DNA-Molekül zu binden.

## Revendications

1. Procédé de détermination de niveaux d'interactions entre biomolécules, telles que des protéines, dans un échantillon, comprenant un premier et un second oligonucléotides porteurs d'informations (IC), le premier et le second oligonucléotides IC étant attachés, de manière covalente ou non covalente, à un premier et second réactifs d'affinité, tels que des anticorps, qui ont la capacité de se lier à une première et une seconde biomolécule, le premier et le second oligonucléotide IC comprenant chacun au moins une partie simple brin qui est complémentaire à une partie d'un oligonucléotide receveur d'informations (IR) ou d'un oligonucléotide activateur, ainsi, après hybridation de l'au moins une partie simple brin dans l'au moins un des premier et second oligonucléotides IC à sa partie complémentaire d'un autre oligonucléotide, permet la mesure de la proportion relative des première et seconde biomolécules interagissantes et non interagissantes dans l'échantillon au niveau d'une seule cellule ou au niveau simple moléculaire, la partie simple brin des premier et second oligonucléotides IC permettant l'interaction
(a) entre le premier et/ou le second oligonucléotide IC et un oligonucléotide récepteur d'informations (IR), l'oligonucléotide (IR) portant au moins deux sites de motif de clivage qui après hybridation à un oligonucléotide IC deviennent double brin pour permettre le clivage, ou
(b) entre le premier et/ou le second oligonucléotide IC et un oligonucléotide activateur, les oligonucléotides IC connectés auxdits anticorps étant conçus pour contenir des structures en épingle à cheveux, et lesdits oligonucléotides IC étant modifiés avec des fluorophores et des désactivateurs, qui sont positionnés de sorte qu'un seul fluorophore par oligonucléotide peut émettre de la lumière, l'hybridation entre le premier et/ou le second oligonucléotide IC et l'oligonucléotide activateur restructurant les positions des fluorophores et des désactivateurs, de telle manière que l'un fluorophore de signalement permet la discrimination entre les biomolécules interagissantes et non interagissantes.

2. Procédé selon la revendication 1, comprenant les étapes consistant à :
a. fournir la molécule d'ADN réceptrice d'informations (IR) simple brin, la molécule d'ADN IR étant circulaire ou linéaire, portant au moins un premier et un second motifs de clivage, les motifs de clivage étant choisis de sorte que les sites de motif de clivage doivent devenir double brin pour permettre le clivage ;
b. fournir la première molécule d'ADN porteuse d'informations (IC), comprenant une partie simple brin qui est complémentaire à la partie de la molécule d'ADN IR portant le premier motif de clivage, l'occurrence de la première molécule d'ADN IC reflétant la quantité d'une première biomolécule dans l'échantillon ;
c. fournir la seconde molécule d'ADN porteuse d'informations (IC), comprenant une partie simple brin qui est complémentaire à la partie de la molécule d'ADN IR portant le second motif de clivage, l'occurrence de la seconde molécule d'ADN IC reflétant la quantité d'une seconde biomolécule dans l'échantillon
d. mélanger les molécules d'ADN des étapes a à c dans des conditions qui permettent la liaison de parties simple brin complémentaires ;
e. ajouter une(des) enzyme(s) de digestion pour créer une (des) coupure(s) au(x) site(s) de motif de clivage qui est/sont devenu(s) double brin, formant ainsi
i. un premier site de liaison d'étiquette de reporter qui permettra la liaison d'une première molécule ou séquence d'ADN d'étiquette de reporter, comprenant une partie simple brin qui est complémentaire à une partie de la première molécule d'ADN IC, et/ou
ii. un second site de liaison d'étiquette de reporter qui permettra la liaison d'une seconde molécule ou séquence d'ADN d'étiquette de reporter comprenant une partie simple brin qui est complémentaire à une partie de la seconde molécule d'ADN IC ;
f. incorporer les séquences d'ADN d'étiquette de reporter par l'une quelconque des alternatives suivantes :
i. ajouter la première molécule d'ADN d'étiquette de reporter et la seconde molécule d'ADN d'étiquette de reporter, moyennant quoi la première molécule d'ADN d'étiquette de reporter est incorporée dans la molécule d'ADN IR au premier site de liaison d'étiquette de reporter si une coupure a été créée au premier site de motif de clivage, et/ou la seconde molécule d'ADN d'étiquette de reporter est incorporée dans la molécule d'ADN IR au second site de liaison d'étiquette de reporter si une coupure a été créée au second site de motif de clivage ; ou
ii. utiliser une ADN polymérase et des nucléotides pour incorporer la première séquence d'ADN d'étiquette de reporter dans la molécule d'ADN IR en remplissant l'espace libre complémentaire au premier site de liaison d'étiquette de reporter sur le premier oligonucléotide IC si une coupure a été créée au premier site de motif de clivage, et/ou incorporer la seconde séquence d'ADN d'étiquette de reporter dans la molécule d'ADN IR en remplissant l'espace libre complémentaire au second site de liaison d'étiquette de reporter sur le second oligonucléotide IC si une coupure a été créée au second site de motif de clivage ; et ajouter une enzyme de ligation liguant la molécule d'ADN IR, fournissant ainsi une molécule d'ADN IR recréée ;
g. éventuellement amplifier la molécule d'ADN IR recréée de l'étape f ;
h. suivre l'incorporation de la première et/ou seconde étiquette(s) de reporter dans la molécule d'ADN IR recréée, comme mesure de l'occurrence et/ou de l'interaction entre la(les) première et/ou seconde biomolécule(s) dans l'échantillon.

3. Procédé selon la revendication 2, dans lequel les motifs de clivage sont choisis parmi des uraciles ou des sites de restriction.

4. Procédé selon la revendication 2 ou 3, dans lequel la(les) enzyme(s) de digestion utilisé(s) pour créer des coupures aux sites de motif de clivage est/sont choisie(s) parmi
i. une endonuléase de coupure en 3' Nb.Bsr.DI coupant en 3' d'un site de restriction double brin,
ii. une endonucléase de coupure en 5' Nt.BsmAI coupant en 5' d'un site de restriction double brin , ou
iii. une combinaison d'uracile-ADN glycolsylase (UDG), enlevant une base uracile à un site double brin, et EndoIV, enlevant le site apyrimidinique.

5. Procédé selon l'une quelconque des revendications 2 à 4, dans lequel le suivi est effectué par
a. fournir un premier oligonucléotide de détection marqué qui est complémentaire à au moins une partie de la première molécule d'ADN d'étiquette de reporter, et un second oligonucléotide de détection marqué qui est complémentaire à au moins une partie de la seconde molécule d'ADN d'étiquette de reporter, et
b. hybrider les premier et second oligonucléotides de détection marqués à la molécule d'ADN IR recréée, qui est éventuellement amplifiée.

6. Procédé selon l'une quelconque des revendications 2 à 5, dans lequel une molécule d'ADN IR recréée linéaire est amplifiée et ensuite séparée par un procédé de séparation, tel qu'une électrophorèse ou une chromatographie, les produits de séparation ayant différentes tailles indiquant l'incorporation d'étiquettes de reporter différentes.

7. Procédé selon l'une quelconque des revendications 2 à 6, dans lequel les identités d'étiquettes de reporter différentes sont suivies par séquençage.

8. Procédé selon l'une quelconque des revendications 2 à 7, dans lequel la molécule d'ADN IR recréée est suivie dans l'échantillon où elle a été formée, tel que par microscopie, ou la molécule d'ADN IR recréée étant collectée de l'échantillon où elle a été formée suivi par le tri et l'analyse de simples molécules, tel que par microscopie.

9. Procédé selon l'une quelconque des revendications 2 à 8, pour enlever des sites abasiques et améliorer l'efficacité de détection, dans lequel les molécules d'étiquette de reporter ont été ajoutées et le premier et/ou second sites d'étiquette de reporter ont/a été incorporés dans l'IR, les étapes suivantes sont effectuées :
i. hybrider une matrice de digestion à la molécule d'ADN IR rendant la surface autour du site abasique restant double brin ;
ii. enlever le site abasique par digestion de la surface double brin par une enzyme de restriction appropriée, telle qu'EndoIV ;
iii. remplir l'espace libre de la surface digérée avec une polymérase appropriée, telle que l'ADN polymérase de T4, pour ajouter la base manquante, telle qu'une thymidine ; et liguer la molécule d'ADN IR avec une ligase, telle que la ligase de T4, pour fournir une molécule d'ADN IR recréée.

10. Procédé selon la revendication 1,
a. dans lequel les premier et second oligonucléotides IC comprennent des structures en épingle à cheveux dans lesquelles des fluorophores et des désactivateurs sont positionnés, les structures en épingle à cheveux étant conçues de sorte qu'un seul fluorophore par oligonucléotide peut émettre de la lumière, et chaque fluorophore ayant un signal unique ;
b. les épingles à cheveux dans les premier et second oligonucléotides IC étant interrompues ou déstabilisées en fournissant un oligonucléotide activateur qui est complémentaire au premier ou au second oligonucléotide IC se liant ainsi au premier ou au second oligonucléotide IC de sorte que le premier et second oligonucléotides IC peuvent interagir l'un avec l'autre entraînant un repositionnement des fluorophores et des désactivateurs ;
c. des paires de conjugués, comprenant des réactifs d'affinité couplés au premier ou second oligonucléotide IC, étant utilisés pour interroger la proximité entre deux biomolécules auxquelles se lient les réactifs d'affinité, un premier profil de signal de fluorophore étant présenté après l'interaction entre la première et seconde biomolécules, et un second profil de signal de fluorophore étant présenté après le manque d'interaction entre la première et la seconde biomolécules, suite à l'hybridation des oligonucléotides l'un à l'autre après l'interaction entre les première et seconde biomolécules entraînant la restructuration des positions des fluorophores et des désactivateurs.

11. Procédé selon l'une quelconque des revendications précédentes, dans lequel les première et seconde biomolécules sont des protéines ou des polypeptides.

12. Procédé selon l'une quelconque des revendications précédentes, dans lequel l'échantillon est un échantillon biologique d'une ou de plusieurs cellules, ou d'un mélange de protéines.

13. Procédé selon l'une quelconque des revendications précédentes, dans lequel la première molécule d'ADN IC est conjuguée à un premier anticorps qui est égal à ou ciblant la première biomolécule, et la seconde molécule d'ADN IC est conjuguée à la seconde molécule d'anticorps étant égale ou ciblant la seconde biomolécule.

14. Procédé selon l'une quelconque des revendications précédentes pour analyser les interactions protéine-protéine dans de simple cellules ou de simples molécules.

15. Kit destiné à être utilisé selon l'une quelconque des revendications 1 à 9, 11 à 14, comprenant
a. une molécule d'ADN réceptrice d'informations (IR) simple brin, portant un premier et un second motif de clivage ;
b. une première et une seconde molécules d'ADN porteuses d'informations (IC), reflétant les quantités d'une première et d'une seconde biomolécules dans un échantillon, la première et la seconde molécules d'ADN IC étant conjuguées à des réactifs d'affinité ou fournies avec des fractions chimiques pour être conjuguées par un utilisateur de kit ;
c. éventuellement des enzymes pour créer des coupures aux sites de motif de clivage dans la molécule d'ADN IR ;
d. une première et une seconde molécules d'ADN d'étiquette de reporter ;
e. éventuellement des réactifs pour l'amplification d'une molécule d'ADN IR recréée ;
f. éventuellement un premier et un second oligonucléotides de détection marqués ; et g. éventuellement une (des) molécule(s) d'ADN et des enzymes pour enlever les sites abasiques.

16. Kit destiné à être utilisé dans le procédé selon les revendications 1, 10 à 14, comprenant
a. une première et une seconde molécules d'ADN porteuses d'informations (IC) comprenant des structures en épingle à cheveux dans lesquelles des fluorophores et des désactivateurs sont positionnés, les structures en épingle à cheveux étant conçues de sorte qu'un seul fluorophore par oligonucléotide peut émettre de la lumière, et chaque fluorophore ayant un signal unique, ayant ainsi la capacité de refléter les quantités d'une première et d'une seconde biomolécules dans un échantillon, les première et seconde molécule d'ADN IC étant conjuguées à un réactif d'affinité ou étant fournies avec des fractions chimiques pour être conjuguées par un utilisateur de kit ; et
b. un oligonucléotide activateur qui est complémentaire à la première ou la seconde molécule d'ADN IC ayant ainsi la capacité de se lier à la première ou la seconde molécule d'IC.
